Europäisches Patentamt

European Patent Office

Office européen des brevets

(19)

(11) Publication number: **0 287 363 B1**

## EUROPEAN PATENT SPECIFICATION

(12)

(45) Date of publication of patent specification: **21.09.94**

(51) Int. Cl.[5]: **C12P 19/06**, C12N 15/52

(21) Application number: **88303352.4**

(22) Date of filing: **14.04.88**

Divisional application 93113193.2 filed on 14/04/88.

(54) **Preparation of xanthan gum.**

(30) Priority: **14.04.87 US 38302**

(43) Date of publication of application:
**19.10.88 Bulletin 88/42**

(45) Publication of the grant of the patent:
**21.09.94 Bulletin 94/38**

(84) Designated Contracting States:
**AT DE FR GB**

(56) References cited:
EP-A- 0 233 019
WO-A-87/05938

**INTERNATIONAL JOURNAL OF BIOLOGICAL MACROMOLECULES; VOL: (; Dec. 1986 pages 372-374;jLondon GB G.C. Barrere et al.:Molecular cloning of genes involved in the production of the extracellular polysaccharide xanthan by Xanthomonas campestris pv. campestris.**

(73) Proprietor: **Shin-Etsu Chemical Co., Ltd.**
**6-1, Otemachi 2-chome**
**Chiyoda-ku Tokyo 100 (JP)**

Proprietor: **SHIN-ETSU BIO, Inc.**
**6650 Lusk Boulevard, Suite B-102**
**San Diego, CA 92121 (US)**

(72) Inventor: **Pollock, Thomas J.**
**4354 Robbins Street**
**San Diego California 92122 (US)**
Inventor: **Thorne, Linda**
**8476 New Salem Street, No. 73**
**San Diego California 92126 (US)**

(74) Representative: **Harrison, David Christopher**
**et al**
**MEWBURN ELLIS**
**York House**
**23 Kingsway**
**London WC2B 6HP (GB)**

ABSTRACTS OF THE ANNUAL MEETING OF THE AMERICAN SOCIETY FOR MICROBIOLOGY, vol. 86 March 26, 1986 page 272 ref. no. 0-64, Washington US, N.E. Harding et al.Genetic and physical analysis of cloned xanthan gum biosynthetic genes from Xanthomonas campestris

JOURNAL OF BACTERIOLOGY, vol 169, no. 8 August 1987, pages 3593-3600 Washington US. L. Thorne et al.:Clustering of mutations blocking synthesis of xanthan gum by Xanthomonas campestris.

APPLIED AND ENVIRONMENTAL MICROBIOLOGY, vol 47, no. 2 Feb. 1984, pages 253-257 Am. Soc. for Microbiol. Washington US, P.M. Walsh et al. "Genetic construction of laxtose-utilizing Xanthomonas capestris"

CHEMICAL ABSTRACTS; VOL:, 79 nol 21 November 26 1973 page 79 ref. no. 122481j, Columbus Ohio US , J. Lucio de Azevedo "Resistance of phytophathogenic bacteria to antibiotics"

**Description**

The present invention relates to the synthesis of xanthan gum by Xanthomonas campestris and particularly to methods for increasing synthesis by modifying the natural organism.

A number of microorganisms produce extracellular polysaccharides, also known as exopolysaccharides or EPS. The exopolysaccharide known as xanthan is produced by the bacterium Xanthomonas campestris. The strain X. campestris pv campestris is a causal agent of black rot of crucifers.

Xanthan itself is useful as a specialty polymer for a growing number of commercial applications. The exploitation of xanthan as a commercial product results from a successful screening effort by the Northern Regional Research Center to find useful water-soluble polysaccharide products to replace existing gums from plant and algal sources. The NRRL discovered X. campestris NRRL B1459, a strain which produces a polymer that exhibits three desirable properties: (1) high viscosity at low concentrations; (2) pseudoplasticity; and (3) insensitivity to a wide range of temperature, pH, and electrolyte concentrations. Because of its special rheological properties, xanthan is used in food, cosmetics, pharmaceuticals, paper, paint, textiles, and adhesives and otherwise in the oil and gas industry.

In addition, the polymer is readily produced by fermentation from D-glucose. The synthesis of xanthan is believed to be similar to exopolysaccharide synthesis by other Gram-negative bacteria, such as species of Rhizobium, Pseudomonas, Klebsiella, and Escherichia. The synthetic pathway can be divided into three parts: (1) the uptake of simple sugars and their conversion to nucleotidal derivatives; (2) the assembly of pentasaccharide subunits attached to an isopentenyl pyrophosphate carrier; and (3) the polymerization of pentasaccharide repeat units and their secretion. By comparison to the more advanced molecular genetic understanding of colanic acid synthesis by E. coli or alginate synthesis by P. aeruginosa, little is known about the genes, enzymes, or mechanisms that control the synthesis of xanthan by X. campestris.

Xanthan gum is usually produced by fermentation of X. campestris with glucose or corn syrup as the major carbon source. Although it is also possible to convert the glucose and galactose in hydrolyzed cheese whey to xanthan gum, wild-type strains of X. campestris utilize lactose poorly, and the whey must first be hydrolyzed enzymatically with lactase or $\beta$-galactosidase. There are some suggestions that the $\beta$-galactosidase of X. campestris has a low affinity for lactose. thereby accounting for the poor utilization of unhydrolyzed lactose. Attempts have been made to generate a strain of X. campestris that can utilize lactose more efficiently. Exogenous lac genes have been transferred into X. campestris using transposon Tn951 which was in turn inserted within the mobilizable broad host range plasmid RP1. However, the plasmid, and therefore the lac genes, were not stable in the absence of a plasmid-selective antibiotic. Other investigators isolated a spontaneous derivative of X. campestris B1459 that could convert unhydrolyzed lactose in whey to xanthan gum. However, the nature of the mutation was not known, and the strain proved to be unstable for xanthan production, losing considerable productivity within forty generations under non-selective conditions.

Other genetic manipulations of X. campestris are also desirable. For example, undesirable enzymes are sometimes produced that contaminate the xanthan product, limiting the usefulness of xanthan gum to a narrower range of situations than would otherwise be possible.

Accordingly, an increased understanding of the genetic control of xanthan production by X. campestris would be useful for improving the productivity of X. campestris for xanthan synthesis.

A recent publication on the topic of molecular cloning of genes involved in the production of xanthan is Barrere et al., Int. J. Biol. Macromol. (1986) 8:372-374. However, Barrere et al made no connection between antibiotic resistance and enhanced xanthan production. A study showing that a mutation, which blocks exopolysaccharide synthesis and prevents nodulation of peas by Rhizobium leguminosarum, was corrected by cloned DNA from the phytopathogen Xanthomonas is described in Borthakur et al., Mol. Gen. Genet. - (1986) 203:320-323. Production of xanthan using Xanthomonas campestris, properties of xanthan, and commercial applications of xanthan are described in Rogovin et al., J. Biochem. Microbiol. Technol. Eng. - (1961) 3:51-63, and Kennedy et al., 1984, "Production, properties, and applications of xanthan", pp. 319-371 in M.E. Bushell (ed.), Progress in Industrial Microbiology, vol. 19, Elsevier, Amsterdam.

A number of publications have occurred after the filing of U.S. Application Serial No. 038, 302 on April 14, 1987 from which priority is claimed in the present application. These include Harding et al., J. Bacteriol. (1987) 169:2854-2861, which describes genetic and physical analyses of a cluster of genes essential for xanthan gum biosynthesis in X. campestris. European Patent Application EP 0 233 019 A2, filed January 29, 1987, describes a recombinant DNA plasmid for xanthan gum synthesis. Thorne et al., J Bacteriol. (1987) 169:3593-3600, describes clustering of mutations blocking synthesis of xanthan gum by X. campestris.

The invention provides a method of increasing xanthan gum production which comprises culturing Xanthomonas campestris strain having a xanthan-increasing modification in a culture medium, wherein said

EP 0 287 363 B1

modification is selected from the group consisting of (1) a mutation causing rifampicin-resistance; (2) a mutation causing bacitracin-resistance; or (3) both; and separating xanthan from the culture medium.

"Resistance" means that the strain is capable of growing in a culture medium containing from 1 $\mu$g/ml rifampicin and/or in a culture medium containing from 100 $\mu$g/ml bacitracin, as the case may be.

The present invention will be better understood by reference to the following detailed description of specific embodiments when considered in combination with the enclosed drawings which form part of the specification, wherein:

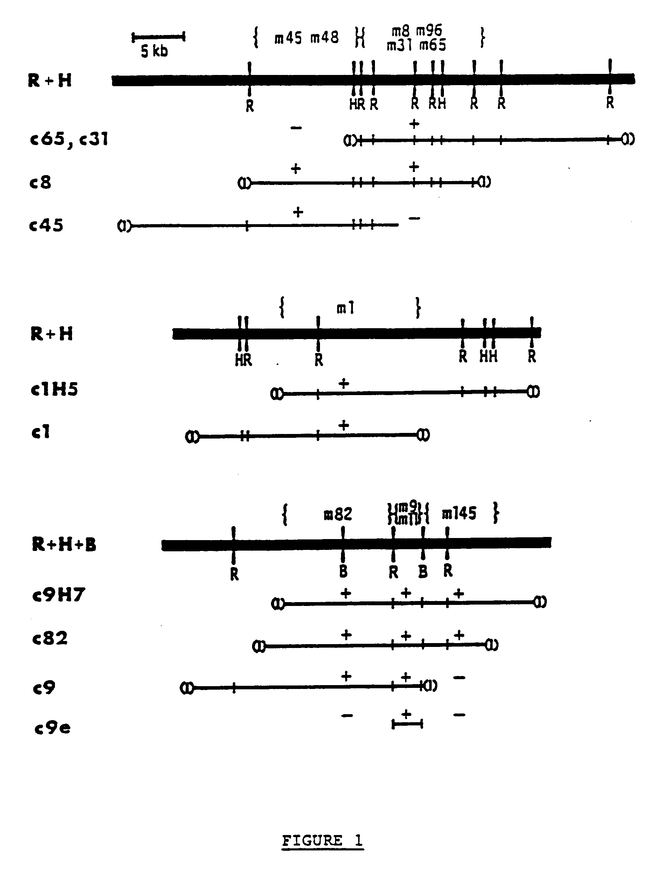

Figure 1 is a compilation of three physical maps for X. campestris DNA insertions in vector pRK311 showing complementation groups. The line marked "R/H" shows the order and position of restriction cleavage sites for EcoRI and HindIII enzymes deduced from the overlapping maps obtained for individual cloned inserts. Parentheses, (), at the end of the cloned maps indicate that it was not possible to distinguish between an end generated by cleavage within the cloned insert from restriction in the adjoining multiple cloning site. The tentative map positions for Xgs mutations are indicated above the physical maps. Un-ordered loci are enclosed with braces, {}.

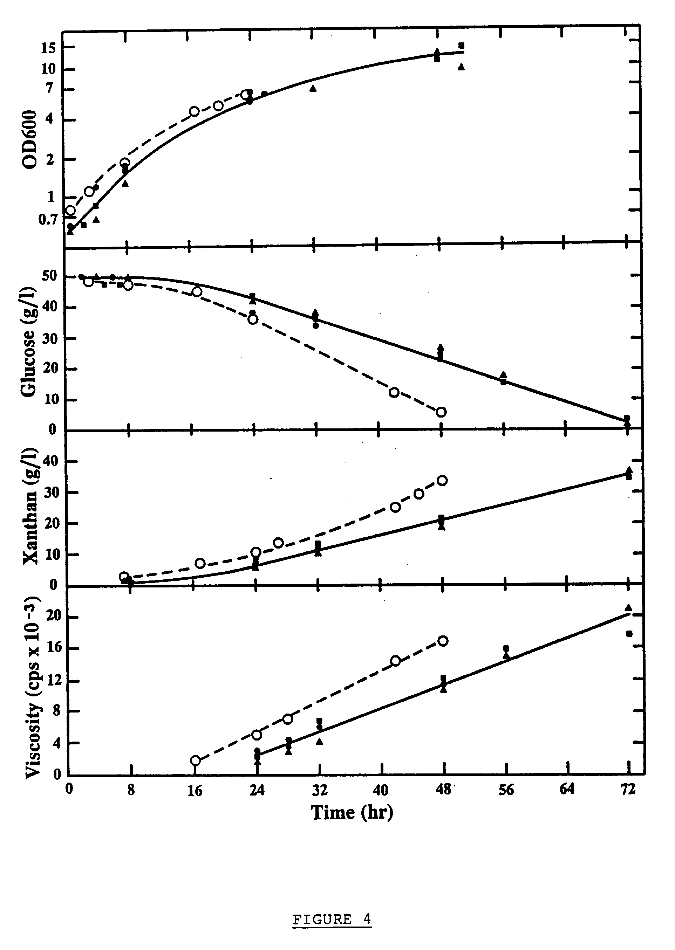

Figure 2 is a graph with four panels showing four different characteristics of three control cultures in comparison to a rifampicin-resistant strain, X59.

DESCRIPTION OF SPECIFIC EMBODIMENTS

Investigations in the laboratories of the inventors have indicated that a number of modifications are available that are capable of increasing xanthan gum production from Xanthomonas campestris strains. Specific genetic modifications capable of increasing xanthan production are mutations causing rifampicin-resistance and mutations causing bacitracin-resistance. A third modification, the presence of exogenous genetic information controlling the synthesis of xanthan introduced into a Xanthomonas campestris strain, is dealt with in divisional application No

The first two of these techniques, both of which involve utilization of a mutant strain having resistance to an antibiotic, can be carried out in a straightforward manner now that the relationship between antibiotic resistance and xanthan production has been determined.

Rifampicin is a member of the group of antibiotics known as rifamycins, produced by Streptomyces mediterraniae. They contain a napthalene ring system bridged between positions 2 and 5 by an aliphatic chain. Rifampicin is known to inhibit DNA-dependent RNA synthesis in prokaryotics, chloroplasts, and mitochondria. Inhibition is due to the formation of a stable complex between RNA polymerase and rifampicin. A description of rifampicin and other rifamycins is set forth in The Concise Encyclopedia of Biochemistry, Walter D. Gruyter, New York, 1983, p. 418.

Bacitracins are branched, cyclic peptides produced by various strains of Bacillus licheniformis. The most important of these peptides is bacitracin A, which contains a thiazoline structure synthesized from an N-terminal isoleucine and its neighboring cystine. The known motive action for bacitracins is by interference with murein synthesis. Murein is a cross-linked polysaccharide-peptide complex of indefinite size that forms a structural constituent of the inner wall layer of all bacteria. Murein consists of linear parallel chains of up to 20 alternating residues of $\beta$-1,4-linked residues of N-acetylglucosamine and N-acetylmuramic acid, extensively cross-linked by peptides.

Resistant mutants can be prepared by culturing a Xanthomonas campestris strain in a culture medium containing one or both of the indicated antibiotics. Antibiotic concentrations of from 1 $\mu$g/ml to 1000 $\mu$g/ml, preferably at least 5 $\mu$g/ml, more preferably at least 50 $\mu$g/ml, preferably no more than 500 $\mu$g/ml, more preferably no more than 250 $\mu$g/ml for rifampicin are useful as initial concentrations in the practice of the present invention. Antibiotic concentrations of from 100 $\mu$g/ml to 1000 $\mu$g/ml, preferably at least 200 $\mu$g/ml, more preferably at least 250 $\mu$g/ml, preferably no more than 500 $\mu$g/ml, more preferably no more than 400 $\mu$g/ml for bacitracin are useful as initial concentrations in the practice of the present invention. These concentrations can be adjusted upward or downward in response to observed conditions of growth and/or survival during cultivation. The remaining components of the culture medium are those normally used for Xanthomonas cultivation and include water, buffering agents such as ammonium phosphate and sodium nitrate, salts such as magnesium sulfate and calcium chloride, glucose sufficient to maintain growth, and trace minerals. Other components such as yeast extract, malt extract, peptone, and Amberex can be utilized if desired.

Selection can be made either for spontaneous mutations that survive growth in the selection media or mutations can be induced by a mutagen such as ultraviolet light or chemical mutagens. Examples of commonly used mutagens are X-rays, ultraviolet radiation at 260nm, N-methyl-N'-nitro-N-nitrosoguanidine, methyl-and ethylmethanesulfonic acid, sodium nitrite, sodium bisulfite, hydroxylamine, nucleic acid base

4

analogs such as 2-aminopurine and 5-bromouracil, and acridine dyes such as proflavin. Also useful are insertional mutations such as insertion sequences, Mu-1 phage, or transposons such as Tn5. A _Xanthomonas campestris_ strain can be exposed to one or more of these mutatens either prior to or concurrently with growth of the strain on the selection medium.

Although not all mutants capable of resisting these two antibiotics show increased xanthan production, the proportion of mutants having increased xanthan production is sufficiently high to allow selection of strains having a xanthan-increasing modification as a result of genetic modification with relative ease. Selection for increased xanthan production can be carried out by measuring xanthan in the culture medium utilizing standard techniques, such as those exemplified in the Examples below. The selected hyperproducing strains can be either utilized as obtained or the genetic information occurring as a result of the mutation can be excised by known techniques of genetic engineering and inserted into other _Xanthomonas_ strains for use in preparing xanthan by culturing techniques. Such genetically engineered strains containing a xanthan-increasing modification that originally arose in a different strain as a result of mutation to give either rifampicin- or bacitracin-resistance fall within the scope of the present invention. The techniques described below in both the general discussion and specific examples of genetic manipulation can be utilized to isolate the genetic information at the locus of the mutation and insert this genetic information into other strains of _Xanthomonas_.

The third specific technique described above for increasing xanthan production involves the use of exogenous genetic information controlling the synthesis of xanthan that has now been identified. Specific sections of _Xanthomonas_ chromosomal DNA have been identified that control the synthesis of xanthan. Figure 1 is a chromosome map providing restriction site information that is useful in identifying the proper sequences. Three deposits of genetic information have also been made (April 10, 1987) with the American Type Culture Collection, Rockville, Maryland, U.S.A., under the Budapest Treaty on the International Recognition of the Deposit of Microorganisms for the Purposes of Patent Procedure. The three deposits are _E. Coli_ strains containing (individually) the three genetic segments identified in Figure 1. These deposits have been accorded deposit numbers ATCC 67386, ATCC 67387, and ATCC 67388.

After a _Xanthomonas_ strain having a xanthan-increasing modification is cultured, xanthan is separated from the culture medium utilizing any technique capable of achieving this result such as the standard techniques already being utilized commercially. See, for example, Kennedy _et al._, _supra_. and Rogovin _et al._, _supra_. One simple technique involves filtering a liquid culture medium to remove growing bacterial cells, adding isopropyl alcohol to the filtrate to precipitate the exopolysaccharides, and collecting the precipitate on a filter followed by drying (optionally with heat and/or under a vaccuum).

The invention now being generally described, the same will be better understood by reference to the following detailed examples which are provided for purposes of illustration only and are not to be considered limiting of the invention unless so stated.

Materials and Methods

Bacterial Strains and Plasmids

_Xanthomonas campestris_ B1459S-4L-II (our strain X55) obtained from the Northern Regional Research Center was the Xgs$^+$ (xanthan gum synthesis positive) parent of all our X. _campestris_ strains. Strain X59 was a spontaneous rifampicin-resistant derivative of X55 that was also fully Xgs$^+$. Rif$^r$ derivatives of X55 arose at a frequencey of about $10^{-9}$ and were selected on agar plates containing Luria broth supplemented with rifampicin at 60 $\mu$g/ml. Bacteriophage $\lambda$ b221 rex::Tn5 cl857 Oam29 Pam80 (Ruvkun _et al._, _Nature_ - (1981) _289_:85-88) was the source of Tn5 for mapping by insertional gene inactivation. Strain LE392 was the permissive host for propagating the phage. All strains and plasmids are listed in Table 1.

## Table 1
### Bacterial Strains and Plasmids

| Name | Genotype or Phenotype[a] | Reference or Source |
|------|--------------------------|---------------------|
| X. campestris | | |
| X55 | Xgs$^+$, prototroph | B1459S-4L-II |
| X59 | Xgs$^+$, prototroph, Rif$^r$ | This Example |
| X59m1 | Xgs$^-$, prototroph, Rif$^r$ | This Example |
| X59m8 | Xgs$^-$, auxotroph, Rif$^r$ | This Example |
| X59m9 | Xgs$^-$, prototroph, Rif$^r$ | This Example |
| X59m11 | Xgs$^-$, auxotroph, Rif$^r$ | This Example |
| X59m31 | Xgs$^-$, prototroph, Rif$^r$ | This Example |
| X59m45 | Xgs$^-$, prototroph, Rif$^r$ | This Example |
| X59m48 | Xgs$^-$, auxotroph, Rif$^r$ | This Example |
| X59m65 | Xgs$^-$, prototroph, Rif$^r$ | This Example |
| X59m82 | Xgs$^-$, prototroph, Rif$^r$ | This Example |
| X59m96 | Xgs$^-$, auxotroph, Rif$^r$ | This Example |
| X59m145 | Xgs-, prototroph, Rif$^r$ | This Example |

6

Table 1 (Cont'd)

Bacterial Strains and Plasmids

| Name | Genotype or Phenotype[a] | Reference or Source |
|---|---|---|
| **E. coli** | | |
| HB101 | $F^-$ hsd20 ($r_B^-$ $m_B^-$), recA13, ara-14, proA2, lacY1, galK2, rpsL20 (streptomycin-resistance), xy1-5, mt1-1, supE44, thi, leu, $\lambda^-$ | Bethesda Research Labs |
| JM109 | recA1, endA1, gyrA96, thi, hsdR17, supE44, relA1, $\Delta$(lac-proAB), [F'traD36, proAB, lacI$^q$Z$\Delta$M15] | Bethesda Research Labs |
| LE392 | $F^-$, hsdR514, ($r_k^-$ $m_k^-$), supE44, supF58, $\lambda^-$, galK2, galT22, metB1, trpR55, lacY1, $\Delta$lac IZY-6 | L. Enquist |
| Bacteriophage | $\lambda$b221 rex::Tn5 (Kan$^r$) cI857, Oam29, Pam80 | Ruvkun et al. Nature (1981) 289:85-88 |
| **Plasmids** | | |
| pRK311 | RK2 origin, Tra$^+$, Mob$^-$, Tet$^r$, $\lambda$cos, lacZ($\alpha$) | Ditta et al. Plasmid (1985) 13:149-153 |
| pRK2013 | ColE1 origin, Imm$^+$, Amp$^r$, Tra$^+$, Mob$^+$, Kan$^r$ | Figurski et al. Proc Natl Acad Sci USA (1979) 76:1648-1652 |
| pUC13 | Amp$^r$, ColE1 origin | Veira et al. Gene (1982) 19:259-268 |
| c1 | pRK311, Tet$^r$, complements m1 | This Example |
| c8 | pRK311, Tet$^r$, complements m8 | This Example |
| c8::Tn5-1$\rightarrow$20 | Tet$^r$, Kan$^r$ | This Example |
| c9 | pRK311, Tet$^r$, complements m9 | This Example |

7

Table 1 (Cont'd)

Bacterial Strains and Plasmids

| Name | Genotype or Phenotype[a] | Reference or Source |
|------|--------------------------|---------------------|
| **Plasmids** (Cont'd) | | |
| c31 | pRK311, $Tet^r$, complements m31 | This Example |
| c45 | pRK311, $Tet^r$ complements m45 | This Example |
| c65 | pRK311, $Tet^r$ complements m65 | This Example |
| c82 | pRK311, $Tet^r$ complements m82 | This Example |
| c1H5 | pRK311, $Tet^r$ complements m1 | This Example |
| c9H7 | pRK311, $Tet^r$ complements m9 | This Example |
| c9e | pRK311, $Tet^r$ complements m9 | This Example |

[a] Abbreviations: $Xgs^+$, xanthan gum synthesis; $Rif^r$, rifampicin resistance; $Tet^r$, tetracycline resistance; $Kan^r$, kanamycin resistance; $Amp^r$, ampicillin resistance; $Imm^+$, colicin E1 immunity; Tra and Mob, transfer and mobilization functions of RK2 plasmid.

Growth Media

Xanthomonas species were cultured by shaking in liquid YT medium at 30°C with rifampicin at 50 $\mu$g/ml, tetracycline at 7.5 $\mu$g/ml and/or kanamycin at 50 $\mu$g/ml added for plasmid maintenance. YT medium contains Bacto tryptone (16 g/l) Bacto yeast extract (10 g/l) and NaCl (5 g/l). All nutrient agar plates contained TBAB (tryptose blood agar base from Difco) plus starch at 1% (w/v). Selection plates for conjugal matings contained tetracycline at 7.5 $\mu$g/ml, kanamycin at 50 $\mu$g/ml and rifampicin at 50 $\mu$g/ml. Minimal agar plates contained M9 inorganic salts (Anderson, Proc. Natl. Acad. Sci. USA (1946) 32:120-128) plus glucose, mannose or fructose at 1% (w/v) as the carbon source. Liquid medium for shake flask experiments to measure xanthan accumulation was referred to as "XG004" and consisted of 1X basic salts, 0.5% (w/v) tryptone, 0.25% (w/v) yeast extract, 1X trace minerals, 0.01% (w/v) CaCl and 2% (w/v) glucose. 10X basic salts consists of 6.8 g $KH_2PO_4$, 0.2 g $MgSO_4 \cdot 7H_2O$, 2.2 g L-glutamic acid, 2 g citric acid in 100 ml with pH adjusted to 7 with NaOH at 30°C. 1000X trace minerals was 2.25 g $FeCl_3 \cdot 6H_2O$, 1.41 g $MnSO_4 \cdot H_2O$, 2.2 g $ZnSO_4 \cdot 7H_2O$, 0.25 g $CuSO_4 \cdot 5H_2O$, 0.4 g $CoCl_2 \cdot 6H_2O$, 0.26 g $Na_2MoO_4 \cdot 2H_2O$, 0.4 g $H_3BO_3$ and 0.06 g KI per liter of deionized $H_2O$ (with HCl added to solubilize the salts). E. coli was grown in Luria broth at 37°C with tetracycline at 10$\mu$g/ml and kanamycin at 50 $\mu$g/ml as appropriate or on agar plates containing Luria broth or TBAB (Difco).

Mutagenesis of X. campestris

About $2 \times 10^9$ freshly grown cells (an absorbance at 600 nm of 1 equals $10^9$ X. campestris cells) were resuspended in 2 ml of minimal salts medium and shaken at 30°C with 0 to 40 μl of ethylmethane sulfonate (EMS) for 1, 2 or 3 h. Samples of 0.5 ml were taken from each treatment, washed two times with YT medium and resuspended in 2 ml of YT medium and shaken overnight at 30°C. Dilutions were spread on TBAB plus 1% (w/v) starch plates. After three days, nonmucoid colonies (about 1% of the total) were saved. The mutants designated X59m1 to X59m150, were tested for retention of the Rif[r] marker of the parent X59, for the presence of cleared zones around colonies on plates containing starch and for ability to utilize different carbon sources.

Example 1

Drug-Resistance and Xanthan Synthesis

Two different mutant phenotypes were associated with elevated accumulation of xanthan gum by Xanthomonas campestris (strain B1459). Among a set of spontaneous rifampicin-resistant mutants of the above strain (designated "X55" in this collection: see Table 1 above), there is a subset that accumulates more xanthan gum in the growth medium. The rifampicin-resistant derivatives that show this unexpected phenotype were X59, X34, X37 and X44. As shown in Table 2 these strains accumulate more xanthan compared to the rifampicin-sensitive parent X55.

A second phenotype. bacitracin-resistance, is also associated with elevated xanthan synthesis. Strain X50 is a bacitracin-resistant derivative of the rifampicin-resistant X59. The double mutant accumulates more xanthan gum than either its parent X59 or X55.

Table 2

Synthesis of Xanthan Gum by

Antibiotic-Resistant X. campestris

| Phenotype | Strain[a] | Experiment Number[b] | | | | | | | | |
| --- | --- | --- | --- | --- | --- | --- | --- | --- | --- | --- |
| | | 1 | 2c | 3c | 4 | 5c | 6c | 7c | 8 | 9 |
| Rif[s] | X55 | 37 | 41 | 38 | 32 | 27 | 119 | 159 | | |
| Rif[r] | X59* | 45 | 46 | 46 | 36 | 36 | 147 | 199 | 177 | 181 |
| | X30 | 39 | | | | | | | | |
| | X31 | 41 | | | | | | | | |
| | X32 | 40 | | | | | | | | |
| | X33 | 39 | 39 | | | | | | | |
| | X34* | 45 | 43 | | | | 149 | 180 | | |
| | X35 | 40 | | | | | | | | |
| | X36 | 39 | | | | | | | | |
| | X37* | 51 | 44 | | | | 143 | 186 | | |
| | X38 | 39 | | | | | | | | |
| | X39 | | | 29 | | | | | | |
| | X40 | | | 30 | | | | | | |
| | X41 | | | 29 | | | | | | |
| | X42 | | | 30 | | | | | | |
| | X43 | | | 30 | | | | | | |
| | X44* | | | 33 | 36 | | 153 | 196 | | |
| | X45 | | | 29 | | | | | | |
| | X46 | | | 29 | | | | | | |
| | X47 | | | 31 | | | | | | |
| | X48 | | | 30 | | | | | | |
| | X49 | | | 34 | 29 | | | | | |
| | RM108* | | | | | | | 182 | | |
| | RM102* | | | | | | | 179 | | |
| | RM109 | | | | | | | 158 | | |
| | RM101 | | | | | | | 157 | | |
| Rif[r]Baci[r] | X50* | | | | | 41 | | | 195 | 192 |

[a] The values in the table are mg of dried precipitate per sample. Strains X30 through X49 were consecutively numbered, random, rifampicin-resistant derivatives of X55. RM108, 102, 109 and 101 were not random isolates. Each overproducer is marked by an asterisk.

[b] For experiments 1-5, the sample size was 8 g of culture broth; for experiments 6-9, the sample size was 10 g. The polysaccharides were precipitated from the sample by adding 2 volumes isopropyl alcohol and mixing. The precipitate was collected by filtration onto a 2.5 cm Whatman 934-AH glass fiber filter and then dried in a vacuum at 80°C and weighed. Culture samples were harvested at 48 hours. The cultures were 20 ml in 250 ml triple-baffled Erlenmeyer flasks. The growth medium for experiment 2 was (per liter of tap water): 1 g $(NH_4)_2HPO_4$,

```
1 g NaNO₃, 1 g Amberex, 0.01 g MgSO₄·7H₂O, 0.1 g CaCl₂,
20 g glucose and 1X trace minerals.  For experiments 1,
3, 4 and 5 the medium was (per liter of tap water):  3 g
yeast extract, 3 g malt extract, 5 g peptone and 20 g
glucose.  For experiments 6 and 7 the medium was (per
liter of tap water):  5 g tryptone, 2.5 g yeast extract,
6.8 g KH₂PO₄, 0.2 g MgSO₄·7H₂O, 2.2 g glutamic acid, 2 g
citric acid, 0.1 g CaCl₂, 20 g glucose and 1X trace min-
erals.  1000X trace minerals were (per liter of deion-
ized water) 2.25 g FeCl₃·6H₂O, 1.41 g MnSO₄·H₂O, 2.2 g
ZnSO₄·7H₂O, 0.25 g CuSO₄·5H₂O, 0.4 g CoCl₂·6H₂O, 0.26 g
Na₂MoO₄·2H₂O, 0.4 g H₃BO₃ and 0.06 g KI.  The medium for
experiments 8 and 9 was (per liter of tap water):  10 g
peptone, 20 g glucose, 3.5 g K₂HPO₄, 2.6 g KH₂PO₄, 0.26
g MgSO₄·7H₂O, 6 mg H₃BO₃, 6 mg ZnO, 2.6 mg FeCl₃·6H₂O,
20 mg CaCO₃ and 0.13 ml 11.6 N HCl.

   c Average values from two independent flasks.
```

The techniques utilized to obtain these resistant strains are described below.

### Rifampicin

At least $10^9$ bacteria of strain X55 were spread on plates (YM plus glucose) containing rifampicin at 50-100 $\mu$g/ml, usually 60 $\mu$g/ml. The cultures were incubated at 30°C for 2-3 days. The colonies that appeared were inspected. Colonies that appeared mucoid ($Xgs^+$) and resistant to rifampicin upon restreaking to purify the mutated derivative were tested for accumulation of xanthan as described in the legend to Table 4 above.

### Bacitracin

To isolate bacitracin-resistance strains, either X55 or the rifampicin-resistant derivative X59 was utilized. The results given in this example are for X59. About $10^9$ bacteria of strain X59 were spread evenly on plates (YM plus glucose) and allowed to dry. Then a drop of a solution containing bacitracin at 1-5 $\mu$g/ml water was spotted on the center of the plate. After 1-2 days of growth at 30°C, a clear zone was present where the bacitracin was added. Just inside the boundary separating the no-growth region from the growth region were several small colonies that survived the antibiotic treatment. These were picked and restreaked on plates (YM plus glucose) containing bacitracin at a concentration of 0.5 mg/ml.

Derivative X50 was obtained from parent X59. Other bacitracin-resistant colonies were seen but were not xanthan producers; such non-mucoid colonies were not studied further.

### Example 2

### Fermentation Conditions

Fermentor inocula were prepared in two growth steps. Four agar plates containing Luria broth were each spread with a loopful of concentrated cells that were stored frozen at -70°C in 15% (v/v) glycerol. When the plates reached confluency (about 48 hrs at 30°C), the cells were harvested by scraping and divided between two 2 l flasks containing 500 ml of Luria broth. The flasks were incubated at 30°C with vigorous shaking for about 16 hrs to yield 10% (v/v) inocula for each strain.

The aerobic "fermentations" were conducted in a Braun Biostat E fermentor using 10 l of the 15 l capacity in either batch or fed-batch mode. The vessel was 430 mm high and 203 mm in diameter and the liquid height was 343 mm. There were 4 Rushton turbine impellers, each with 6 flat blades. For batch fermentation the non-optimized medium contained (per liter of tap water): 50 g (glucose equivalents) corn syrup (CPC or Hubinger), 1 g Amberex 510 (Universal Foods Corp.), 1 g $(NH_4)_2HPO_4$, 1 g $NaNO_3$, 0.1 g $CaCl_2$, 0.01 g $MgSO_4 \cdot 7H_2O$ and 1 ml 1000X trace elements. The latter is comprised of (per liter of deionized water): 2.25 g $FeCl_3 \cdot 6H_2O$, 2.2 g $ZnSO_4 \cdot 7H_2O$, 1.41 g $MnSO_4 \cdot H_2O$, 0.4 g $CoCl_2 \cdot 6H_2O$, 0.4 g

$H_3BO_3$, 0.26 $Na_2MoO_4 \cdot 2H_2O$, 0.25 g $CuSO_4 \cdot 5H_2O$ and 0.06 g KI. The pH was maintained at 7 by incremental addition of 2.5 N NaOH or 0.5 N HCl. Dissolved oxygen was regulated at 60% by air flow variation from 0.5-20 l/min and agitation speeds between 300 and 1000 rpm. The fed-batch fermentations were as above, but with these changes. The medium consisted of the following per liter of tap water: 15 g peptone or yeast extract, 3.5 g $K_2HPO_4$, 2.6 g $KH_2PO_4$, 0.26 g $MgSO_4 \cdot 7H_2O$, 6 mg $H_3BO_3$, 6 mg ZnO, 2.6 mg $FeCl_3 \cdot 6H_2O$ and 20 mg $CaCO_3$. The pH was adjusted to 7.0 with 2.5 N NaOH. The feeding medium was the same but without the peptone and was 6X concentrated. The feed was pumped into the vessel at a rate of 60-100 ml/hr.

Analytical Procedures

The amount of xanthan accumulated in the growth medium was determined by weighing a sample of about 10 ml and precipitating the polysaccharides with two volumes of isopropyl alcohol. The precipitate was collected on a glass filter (Whatman® 934-AH), dried in a vacuum at 80°C and weighed. For viscosity measurements the dried precipitate was ground in a mortar and sieved through a 250 micron mesh before resuspending in a 0.1% (w/v) NaCL. Viscosity measurements over a range of shear rates at room temperature were made with a Brookfield LVT viscometer. Protein concentrations were determined with the "BioRad Protein Assay" and standards of bovine serum albumin (Sigma).

Fermentation of Mutants of X. campestris at the 10 l Scale

Two modes of fermentation were carried out: batch and fed-batch. We did not rigorously optimize the culture conditions for either mode. The batch mode conditions were adopted from several reports in the literature. The results of four fermentations are given in Figure 2. Three control cultures (X55, ● ; X56, ▲ ; X57, ■ ) were compared to the rifampicin-resistant X59, 0. Strains X56 and X57 are X. campestris NRRL-B1459 obtained from the Northern Regional Research Center and the American Type Culture Collection (ATCC 13951), respectively. As shown in the four panels in Figure 4, the three controls were not distinguishable for cell density, glucose consumed, xanthan produced or culture viscosity. The mutant strain, X59, was different in three respects. First, the rate of consumption of glucose is 1.4 times faster for X59 for the time interval beginning at about 20 hrs and extending until the end of the fermantation. Second, for this interval, xanthan accumulation is about 1.5 times higher for X59. Third, the viscosity of the X59 fermentation broth is about 1.6 times higher than the three controls. These three differences are of similar magnitudes. We believe that the improvement in productivity for strain X59 reflects a more efficient conversion of the substrate glucose to xanthan gum rather an effect on cell growth rates or final cell mass.

The two strains X59 and X50 were also grown in fed-batch mode with an improved medium. Modifications of conditions were initially tested at the shake flask level. The results of the fed-batch fermentations are summarized in Table 3. The nitrogen source was either yeast extract or peptone (casein). Feeding was with a glucose plus salts solution such that glucose averaged about 25 g/l, but ended at about 5-10 g/l. As seen earlier in shake flask experiments, the bacitracin-resistant derivative of X59 accumulated more xanthan than its parent. No attempt was made to optimize the culture conditions for strain X50.

Table 3

| Fed-batch Fermentations | | | | | | |
|---|---|---|---|---|---|---|
| Strain: | | X59 | | | | X50 |
| Medium: | | Yeast Extract | | Peptone | | Peptone |
| Hours: | 48 | 61 | 48 | 63 | 48 | 71 |
| Absorbance (600 nm) | 11 | 12 | 17 | 15 | 15 | 15 |
| Xanthan (g/l) | 47 | 59 | 49 | 58 | 52 | 66 |
| Viscosity (cps x $10^{-3}$) | 27 | 38 | 37 | 47 | 33 | 45 |
| Yield (g xanthan/g glucose) | -- | 0.80 | -- | 0.85 | -- | 0.85 |
| Global productivity (g xanthan/l/h) | 0.98 | 0.96 | 1.04 | 0.92 | 1.07 | 0.92 |

**Claims**

1. A method for producing xanthan gum, comprising:
   culturing, in a culture medium, a pure culture of a Xanthomonas campestris strain which is a spontaneous or induced mutant derived from a parent strain, which pure culture strain is rifampicin and/or bacitracin resistant and produced more xanthan than said parent strain, "resistant" meaning that the strain is capable of growing in a culture medium containing from 1 $\mu$g/ml rifampicin and/or in a culture medium containing from 100 $\mu$g/ml bacitracin, as the case may be.

2. A method according to claim 1 which further comprises separating the xanthan gum produced from the culture medium.

3. A method according to claim 1 or claim 2, wherein said strain is capable of producing at least 1 gram of xanthan per liter of culture medium per hour.

4. A pure culture of a Xanthomonas campestris strain which is a spontaneous or induced mutant derived from a parent strain, which pure culture strain is rifampicin and/or bacitracin resistant, "resistant" meaning that the strain is capable of growing in a culture medium containing from 1 $\mu$g/ml rifampicin and/or in a culture medium containing from 100 $\mu$g/ml bacitracin, as the case may be, and produces more xanthan than said parent strain.

5. A method for isolating a mutant strain of Xanthomonas campestris according to claim 4, comprising:
   a) selecting rifampicin resistant mutants from said parental strain by growing said parental strain on medium containing rifampicin and/or bacitracin;
   b) measuring the xantham gum production levels of said selected resistant mutants; and
   c) selecting those resistant mutants which produce xanthan gum in excess to that produced by said parental strain.

6. A method according to claim 5, wherein prior to step a), said parental strain is subjected to induced mutagenization.

**Patentansprüche**

1. Verfahren zur Herstellung von Xanthangummi, umfassend:
   das Kultivieren einer reinen Kultur eines Xanthomonas campestris-Stammes, der ein von einem Elternstamm abgeleiteter soontaner oder induzierter Mutant ist, in einem Kulturmedium, welcher reine Kulturstamm rifampicin- und/oder bacitracinresistent ist und mehr Xanthan erzeugt als der genannte Elternstamm, wobei "resistent" bedeutet, daß der Stamm je nach Fall zum Wachstum in einem Kulturmedium, das ab 1 $\mu$g/ml Rifampicin enthält, und/oder in einem Kulturmedium, das ab 100 $\mu$g/ml Bacitracin enthält, fähig ist.

2. Verfahren nach Anspruch 1, das weiters das Abtrennen des erzeugten Xanthangummis aus dem Kulturmedium umfaßt.

3. Verfahren nach Anspruch 1 oder 2, worin der genannte Stamm zur Herstellung von zumindest 1 g Xanthan pro Liter Kulturmedium und Stunde fähig ist.

4. Reine Kultur eines Xanthomonas campestris-Stammes, der ein von einem Elternstamm abgeleiteter spontaner oder induzierter Mutant ist, welcher reine Kulturstamm rifampicin- und/oder bacitracinresistent ist, wobei "resistent" bedeutet, daß der Stamm je nach Fall zum Wachstum in einem Kulturmedium, das ab 1 $\mu$g/ml Rifampicin enthält, und/oder in einem Kulturmedium, das ab 100 $\mu$g/ml Bacitracin enthält, fähig ist, und mehr Xanthan erzeugt als der genannte Elternstamm.

5. Verfahren zum Isolieren eines Mutantenstammes von Xanthomonas campestris nach Anspruch 4, umfassend:
   a) das Auswählen rifampicinresistenter Mutanten aus dem genannten Elternstamm durch Züchtung des genannten Elternstammes auf Medium, das Rifampicin und/oder Bacitracin enthält:

EP 0 287 363 B1

b) das Messen der Xanthangummiproduktionsmengen der genannten ausgewählten resistenten Mutanten; und

c) das Auswählen derjenigen resistenten Mutanten, die Xanthangummi in größerem Ausmaß erzeugen als der genannte Elternstamm.

6. Verfahren nach Anspruch 5, worin der genannte Elternstamm vor Schritt a) einer induzierten Mutagenisierung unterzogen wird.

**Revendications**

1. Méthode pour produire de la gomme xanthane, comprenant :

la culture, dans un milieu de culture, d'une culture pure d'une souche de Xanthomonas campestris qui est un mutant spontané ou induit dérivé d'une souche parente, laquelle souche de culture pure est résistante à la rifampicine et/ou à la bacitracine et produit plus de xanthane que ladite souche parente, "résistante" signifiant que la souche est capable de croître dans un milieu de culture contenant à partir de 1μg/ml de rifampicine et/ou dans un milieu de culture contenant à partir de 100μg/ml de bacitracine, comme cela peut être le cas.

2. Méthode selon la revendication 1 qui comprend de plus la séparation de la gomme xanthane produite du milieu de culture.

3. Méthode selon la revendication 1 ou 2, dans laquelle ladite souche est capable de produire au moins 1 gramme de xanthane par litre de milieu de culture par heure.

4. Culture pure d'une souche de Xanthomonas campestris qui est un mutant induit ou spontané dérivé d'une souche parente, laquelle souche de culture pure est résistante à la rifampicine et/ou la bacitracine, "résistante" signifiant que la souche est capable de croître dans un milieu de culture contenant à partir de 1μg/ml de rifampicine et/ou dans un milieu de culture contenant à partir de 100μg/ml de bacitracine, comme cela peut être le cas, et produit plus de xanthane que ladite souche parente.

5. Méthode pour isoler une souche mutante de Xanthomonas campestris selon la revendication 4, comprenant :

a) sélection de mutants résistants à la rifampicine à partir de ladite souche parente par croissance de ladite souche parente sur un milieu contenant de la rifampicine et/ou de la bacitracine;

b) mesure des niveaux de production de gomme xanthame desdits mutants résistants choisis; et

c) sélection de ces mutants résistants qui produisent de la gomme xanthane en excès à celle produite par ladite souche parente.

6. Méthode selon la revendication 5, dans laquelle avant l'étape a), ladite souche parente est soumise à une mutagénisation induite.

14

FIGURE 1

FIGURE 4